# EUROPEAN PATENT SPECIFICATION

(11) **EP 4 554 528 B1**
(45) Date of publication and mention of the grant of the patent: **09.12.2026**
(21) Application number: 23744670.3
(22) Date of filing: 13.07.2023
(51) Int. Cl.: A61F 5/445

(54) **OSTOMY APPLIANCE WITH ADHESIVE HAVING TROUGH AND CREST PORTIONS**
OSTOMIEVORRICHTUNG MIT KLEBSTOFF MIT TROG- UND SCHEITELTEILEN
APPAREIL DE STOMIE À ADHÉSIF AYANT DES PARTIES DE CREUX ET DE CRÊTE

(30) Priority: 14.07.2022 DK PA202270377
(43) Date of publication of application: 21.05.2025
(73) Proprietor: Coloplast A/S, 3050 Humlebaek (DK)
(72) Inventor: MOLZEN, Lars, 3450 Alleroed (DK); SUND, Anders Grove, 3450 Alleroed (DK)
(86) International application number: PCT/DK2023/050185
(87) International publication number: WO 2024/012643

(56) References cited:
- WO-A1-2019/120429
- WO-A1-2019/120448
- US-A1- 2021 000 635

## Description

### TECHNICAL FIELD

The present disclosure relates to an ostomy appliance and a method of manufacturing such ostomy appliance.

### BACKGROUND

Stomal output often contains body fluids and visceral contents that are aggressive to both the skin of a user and to ostomy devices. These have a detrimental effect on the efficiency and integrity of the adhesive materials that are applied to attach the ostomy device to the user's skin surface. For users in general safe, reliable and efficient ostomy devices are evidently highly desirable.

However, a particularly major and persistent concern of a large population of ostomists continues to be failure of the base plate adhesive attaching the ostomy appliance to the user's skin surface, because such failure almost inevitably leads to embarrassing and stigmatising leakage incidents. Such incidents in turn are known from several user interviews to lead to a reduced quality-of-life feeling. Adhesive failure of the base plate adhesive can result from various reasons. Most often, a leakage incident is caused by stomal output entering between the proximal surface of the base plate and the user's skin, e.g. due to less-than-optimal attachment of the base plate to the skin arising from e.g. uneven skin surface or skin folds. This undesirable progression of stomal output "underneath" the adhesive leads to deterioration and/or weakening of the adhesive material carrying the weight and providing the seal of the ostomy appliance. Often, such failure happens surprisingly fast and is only detectable for the user once the failure has already become so severe that leakage occurs, requiring immediate change of the ostomy appliance and possibly also of the user's clothes.

In other instances, the primary factor of adhesive failure is simply a question of how much time has elapsed since the base plate of the ostomy appliance was first applied to the user's skin surface. In addition to the output from the stoma itself, the peristomal skin surface continuously secretes some moisture (e.g. sweat). To mitigate this, most often adhesives of base plates for ostomy devices include hydrocolloid materials which are capable of absorbing high levels of moisture, thereby stabilizing the polymer matrix of the adhesive material and prolonging the lifetime ("wear time") of the base plate. However, eventually the adhesion capability of the base plate no longer can support the force exerted on the base plate from the load of the output collecting bag, and the appliance must be replaced.

As there can be considerable differences in the severity and/or speed by which adhesive failure and potentially leakage occur, which differences at least to some extent are correlated to various factors including those presented above, a mere indication that failure or leakage is imminent, or that it has already occurred, fails to represent a reliable and satisfactory solution to the problem of avoiding sudden embarrassing and stigmatising leakage incidents in ostomy appliances. In other words, the users of ostomy appliances could greatly benefit from an appliance solution which provides them with better guidance and options regarding how and - not least - how quickly to react to beginning failure or leakage of the adhesive of the base plate of the appliance. More generally, ostomists and health care professionals alike would welcome improvements in ostomy devices to reduce or eliminate the occurrence of sudden leakage incidents.

WO 2019/120448 A1 relates to a system for preserving functionality of a skin-engageable element for attachment to a skin portion of a user during storage including a skin engageable element and a protective element having discrete portions projecting therefrom. The discrete portions are adapted to engage with through-going passages in an adhesive layer of the skin-engageable element, and ensure that the passages are open and passable when the protective element is removed before use of the skin-engageable element.

### SUMMARY

On this background, it may be seen as an object of the present disclosure to provide an ostomy appliance, such as a sensor patch and/or a base plate, for facilitating reliable and/or improved detection of risk of failure of an ostomy appliance and/or improved detection of risk of leakage.

Another object of the present disclosure is to provide a method of manufacturing such an ostomy appliance.

One or more of these objects may be met by aspects of the present disclosure as described in the following.

A first aspect of the present disclosure relates to an ostomy appliance as set out in claim 1.

By covering electrode paths by a relatively thin portion of the adhesive layer, liquid or moisture, such as stomal output, may quickly propagate through the adhesive to give off a signal indicative of liquid in the interface between the skin surface and the ostomy appliance. Thus, if liquid, such as stomal output, is present between the one or more trough portions and the skin surface of the user, a signal indicative of this can be obtained from the first and second electrode paths. The present ostomy appliance advantageously allows for providing a coherent/integral adhesive layer since a good sensor signal can be obtained through the relatively thin trough portions. Furthermore, the relatively thick crest portion can function as a moisture reservoir so that the lifetime of the adhesive layer is not adversely affected by the thin trough portions.

In some embodiments, the plurality of electrode paths comprises a number of discrete electrode measurement points (i.e. without any openings in the adhesive materials and without respective protrusions of the release liner). Accordingly, the ostomy appliance may comprise a masking layer electrically insulating the electrode paths between the number of discrete electrode measurement points.

The one or more trough portions extend circumferentially at least partly, but preferably entirely, around the stomal opening, and the crest portion extends circumferentially at least partly, but preferably entirely, around the stomal opening.

Additionally, the distal surface of the adhesive layer may be substantially flat and may be arranged on the proximal surface of the support layer. The circumferential crest portion and the one or more circumferential trough portions at least partly provide the proximal surface of the adhesive layer with a ripple shape extending radially from the centre point. Alternatively, the proximal surface of the adhesive layer may be substantially flat, and the distal surface of the adhesive layer may be arranged on the proximal surface of the support layer. Here, the circumferential crest portion and the one or more circumferential trough portions at least partly provide the distal surface of the adhesive layer with a ripple shape extending radially from the centre point, whereby a ripple shape may thus also be formed or reflected in the support layer. In embodiments, the support layer, the plurality of electrode paths, and the adhesive layer may be of such a flexible nature and/or have such a thickness that the trough and crest portions, and any resulting ripple shape, adapt naturally to the skin when worn and thus may be considered substantially flat relative to the skin irrespective of the relative shape between the support layer and the adhesive layer discussed above.

Additionally or alternatively, the crest portion may be arranged on a radially outer side of the one or more trough portions. Preferably, the crest portion may be arranged on a radially outer side of a first trough portion of the one or more trough portions. The first trough portion may cover the first electrode path and/or second electrode path.

Additionally or alternatively, the crest portion may be arranged on a radially inner side of the one or more trough portions. The crest portion may be arranged adjacent to the stomal opening.

Additionally or alternatively, the crest portion may be arranged between a first trough portion and a second trough portion. The first trough portion may cover the first electrode path and the second trough portion may cover the second electrode path.

Additionally or alternatively, the thickness of the one or more trough portions may be at most 150 µm, preferably at most 100 µm. Alternatively, the thickness of the one or more trough portions may be in the range from 50 to 150 µm but may more preferably be in the range from 80 to 100 µm. The thicknesses may apply to one or both of the first and second trough portions. The lower end of the ranges for the thickness of the one or more trough portions may be bound by a grain size of hydrocolloids particles included in the adhesive layer.

Additionally or alternatively, the thickness of the crest portion may be at least 80 µm, preferably at least 100 µm, more preferably at least 150 µm, even more preferably at least 200 µm.

Additionally or alternatively, the difference between the thickness of the one or more trough portions and the thickness of the crest portion may be at least 30 µm, preferably at least 50 µm, more preferably at least 100 µm.

Additionally or alternatively, the first and second electrode paths, preferably each electrode path of the plurality of electrode paths, may comprise two end points. A first end point may be arranged in a neck portion of the ostomy appliance and a second end point may be covered by the respective trough portion of the adhesive layer. For example, the plurality of electrode paths may form loops and/or open loops. Open loop electrode path(s) may enable electrode arrangement in a few or a single electrode layer.

Additionally or alternatively, the plurality of electrode paths may extend circumferentially at least partly, but preferably substantially entirely, around the stomal opening.

The first and the second electrode paths may form a first sensor. The plurality of electrode paths may further comprise a ground electrode path, a third electrode path, a fourth electrode path, a fifth electrode path and/or a sixth electrode path. The first electrode path may be a common ground electrode (which may also be denoted a reference electrode path or a common electrode path). For example, a second sensor may be formed by the first electrode path and the third electrode path, a third sensor may be formed by the first electrode path and the fourth electrode path, a fifth sensor may be formed by the first electrode path and the fifth electrode path, and/or a sixth sensor may be formed by the first electrode path and the sixth electrode path. Each electrode path may have respective connection parts for connecting the electrode paths to respective terminal elements of a monitor device. The respective connection parts may be arranged in the neck portion of the ostomy appliance.

Additionally or alternatively, the first and second electrode paths may be configured for allowing the provision of a signal indicative of liquid on the proximal surface of the adhesive layer. Alternatively, the first and second electrode paths may be configured for allowing the provision of a signal indicative of a leak between the skin of a user and the support layer, preferably between the skin of a user and the proximal surface of the adhesive layer.

The plurality of electrode paths is electrically conductive and may comprise one or more of metallic (e.g. silver, copper, gold, titanium, aluminium, stainless steel), ceramic (e.g. ITO), polymeric (e.g. PEDOT, PANI, PPy), and carbonaceous (e.g. carbon black, carbon nanotube, carbon fibre, graphene, graphite) materials.

Additionally or alternatively, the plurality of electrode paths may further include a third electrode path and a fourth electrode path. The third and fourth electrode paths may be configured for allowing the provision of a signal indicative of the moisture content of the adhesive layer. The crest portion may be arranged to cover the third and fourth electrode paths.

Additionally or alternatively, a first trough portion of the one or more trough portions may be delimited by the first electrode path and the second electrode path. Alternatively, the first trough portion of the one or more trough portions may be delimited by a radially innermost electrode path and a radially outermost electrode path of the plurality of electrode paths.

The support layer may also be known as a substrate.

Additionally or alternatively, the support layer may preferably be moisture permeable. Alternatively, the support layer is moisture impermeable.

Additionally or alternatively, the support layer may be a non-woven material and/or a perforated polymer-film material.

Additionally or alternatively, the ostomy appliance may comprise a release liner arranged on the proximal surface of the adhesive layer.

Additionally or alternatively, the ostomy appliance may comprise a base plate. The base plate may include the support layer, the plurality of electrode paths, and the adhesive layer. In this embodiment, the ostomy appliance may thus be a one-piece ostomy appliance or a two-piece ostomy appliance. The one-piece ostomy appliance comprises, in addition to the base plate, an ostomy pouch fixedly attached to the base plate. The two-piece ostomy appliance comprises, in addition to the base plate, an ostomy pouch detachably attached to the base plate. For example, the base plate and the ostomy pouch may be releasably coupled e.g. with a mechanical and/or an adhesive coupling, e.g. to allow that a plurality of ostomy pouches can be utilized (exchanged) with one base plate. For example, the base plate may comprise a coupling ring for coupling an ostomy pouch to the base plate. Further, a two-piece ostomy appliance may facilitate correct application of the base plate to skin, e.g. to an improved user sight of the stomal region. The base plate may be configured for coupling to a user's stoma and/or skin surrounding the stoma, such as a peristomal skin area.

Alternatively, the ostomy appliance may comprise a sensor patch. The sensor patch may include the support layer, the plurality of electrode paths, and the adhesive layer. The sensor patch may further have a proximal side and a distal side. The distal side may be adapted for attachment to an adhesive surface of a base plate of the ostomy appliance. The proximal side may be adapted for attachment to a skin surface of a user. In this embodiment, the ostomy appliance may be a one-piece ostomy appliance or a two-piece ostomy appliance. The one-piece ostomy appliance comprises a base plate as well as an ostomy pouch fixedly attached to the base plate in addition to the sensor patch, which may be provided separately. The two-piece ostomy appliance comprises a base plate with a detachable ostomy pouch in addition to the sensor patch, which may be provided separately. For example, the base plate and the ostomy pouch may be releasably coupled e.g. with a mechanical and/or an adhesive coupling, e.g. to allow that a plurality of ostomy pouches can be utilized (exchanged) with one base plate. For example, the base plate may comprise a coupling ring for coupling an ostomy pouch to the base plate. Further, a two-piece ostomy appliance may facilitate correct application of the base plate to skin, e.g. to an improved user sight of the stomal region.

A second aspect of the present disclosure relates to a method of manufacturing an ostomy appliance as set out in claim 15.

A person skilled in the art will appreciate that any one or more of the above aspects of this disclosure and embodiments thereof may be combined with any one or more of the other aspects of this disclosure and embodiments thereof.

### BRIEF DESCRIPTION OF THE DRAWINGS

Embodiments of this disclosure will be described in more detail in the following with regard to the accompanying figures. The figures show one way of implementing the present invention and are not to be construed as being limiting to other possible embodiments falling within the scope of the attached claim set.
Fig. 1 schematically illustrates an exploded view of an exemplary two-piece ostomy appliance.
Fig. 2 schematically illustrates an exploded view of an exemplary two-piece ostomy appliance comprising a sensor patch.
Fig. 3 schematically illustrates an exemplary electrode path configuration.
Fig. 4 schematically illustrates a perspective view of an exemplary ostomy appliance with the support layer omitted for visualization purposes. The ostomy appliance has been cut to illustrate the cross-section of an adhesive layer for adhering to the skin of the user.
Fig. 5 schematically illustrates the same view as Fig. 4 but of an alternative embodiment of an ostomy appliance.

### DETAILED DESCRIPTION

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with respect to the orientation of the Figure(s) being described. Because components of embodiments can be positioned in a number of different orientations, the directional terminology is used for purposes of illustration and is in no way limiting. It is to be understood that other embodiments may be utilized, and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other, unless specifically noted otherwise.

Throughout this disclosure, the words "stoma" and "ostomy" are used to denote a surgically created opening bypassing the intestines or urinary tract system of a person. The words are used interchangeably, and no differentiated meaning is intended. The same applies for any words or phrases derived from these, e.g. "stomal", "ostomies" etc. Also, the solid and liquid wastes emanating from the stoma may be referred to as both stomal "output", "waste(s)", and "fluids" interchangeably. A subject having undergone ostomy surgery may be referred to as "ostomist" or "ostomate" - moreover, also as "patient" or "user". However, in some cases "user" may also relate or refer to a health care professional (HCP), such as a surgeon or an ostomy care nurse or others. In those cases, it will either be explicitly stated or be implicit from the context that the "user" is not the "patient" him- or herself.

In the following, whenever referring to proximal side of a device or part of a device, the referral is to the skin-facing side, when the ostomy appliance is worn by a user. Likewise, whenever referring to the distal side of a device or part of a device, the referral is to the side facing away from the skin, when the ostomy appliance is worn by a user. In other words, the proximal side is the side closest to the user, when the appliance is fitted on a user and the distal side is the opposite side - the side furthest away from the user in use.

The axial direction is defined as the direction of the stoma, when the appliance is worn by a user. Thus, the axial direction is generally perpendicular to the skin or abdominal surface of the user.

The radial direction is defined as transverse to the axial direction that is transversely to the direction of the stoma, i.e. "across" the distal/proximal surface of the base plate. In some sentences, the words "inner" and "outer" may be used. These qualifiers should generally be perceived with respect to the radial direction, such that a reference to an "outer" element means that the element is farther away from a centre portion of the ostomy appliance than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do not provide the effect.

The use of the word "essentially" as a qualifier to certain structural and functional features or effects in this disclosure is used for emphasizing what is the most important focus of something or fact about something (i.e. a feature may have or fulfil a variety of effects, but when the disclosure discusses one effect as being "essentially" provided, this is the focus and the most important effect in relation to the disclosure).

Throughout the disclosure, the use of the terms "first", "second", "third", "fourth", "primary", "secondary", "tertiary" etc. does not imply any particular order or importance but is included merely to identify individual elements. Furthermore, the labelling of a first element does not imply the presence of a second element and vice versa.

Disclosed is an ostomy appliance which can either be a one-piece ostomy appliance or a two-piece ostomy appliance. The ostomy appliance comprises a base plate and an ostomy pouch (also referred to as an ostomy bag). The base plate is configured for coupling to a user's stoma and/or skin surrounding the stoma, such as a peristomal skin area. The ostomy appliance may be a colostomy appliance, an ileostomy appliance or a urostomy appliance. The ostomy appliance may be a two-piece ostomy appliance, i.e. the base plate and the ostomy pouch may be releasably coupled e.g. with a mechanical and/or an adhesive coupling, e.g. to allow that a plurality of ostomy pouches can be utilized (exchanged) with one base plate. For example, the base plate may comprise a coupling ring for coupling an ostomy pouch to the base plate. Further, a two-piece ostomy appliance may facilitate correct application of the base plate to skin, e.g. to an improved user sight of the stomal region. Alternatively, the ostomy appliance may be a one-piece ostomy appliance, i.e. the base plate and the ostomy pouch may be fixedly attached to each other. The one-piece or two-piece ostomy appliance may further include a separate sensor patch for attachment to the base plate. The sensor patch may facilitate detection of moisture propagation in an adhesive material as well as detection of a heightened risk of leakage. For example, the sensor patch may allow electronic measurements of performance of the base plate and/or to facilitate detection of increasing risks of leakage and/or to facilitate detection of decreasing adherence of the base plate to the skin of the user.

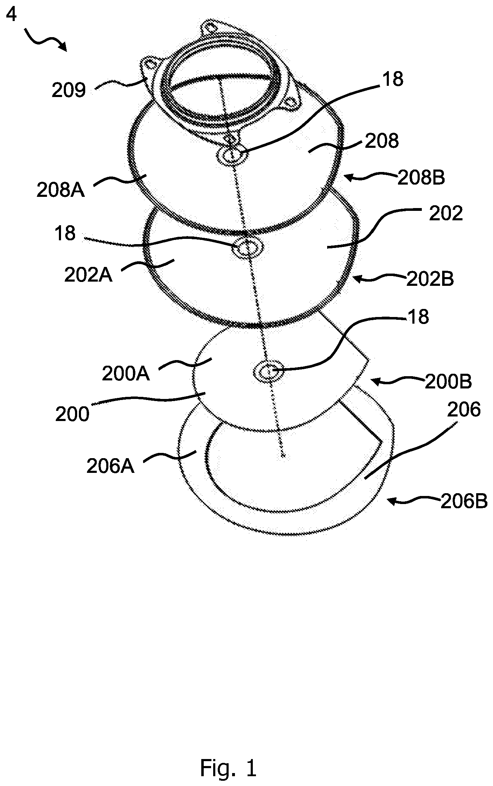

Fig. 1 schematically illustrates an exploded view of an exemplary a two-piece ostomy appliance comprising a base plate 4. The base plate 4 comprises a first adhesive layer 200 having a distal surface 200A and a proximal surface 200B. During use, the proximal surface 200B of the first adhesive layer 200 adheres to the user's skin. The base plate 4 comprises a second adhesive layer 202 having a distal surface 202A and a proximal surface 202B. As illustrated, the second adhesive layer 202 spans a larger surface area than the first adhesive layer 200, such as to provide a rim of the proximal surface 202B of the second adhesive layer 202 surrounding the proximal surface 200B of the first adhesive layer 200.

The base plate 4 comprises a release liner 206, which may be peeled off by the user prior to applying the base plate 4 to the skin. The release liner 206 comprises a distal surface 206A and a proximal surface 206B. The distal surface 206A of the release liner 206 is covering the proximal surface of the first adhesive layer 200 and covering the proximal surface of the second adhesive layer 202 not covered by the first adhesive layer 200.

The base plate 4 comprises a backing layer 208. The backing layer 208 is a protective layer protecting the adhesive layers, such as the first adhesive layer 200 and/or the second adhesive layer 202 from external strains and stress during use. Furthermore, the backing layer 208 also covers the adhesive layers, such as the first adhesive layer 200 and/or the second adhesive layer 202, such that the adhesive layers 200, 202 do not adhere to clothes worn on top of the base plate 4. The backing layer 208 comprises a distal surface 208A and a proximal surface 208B. The distal surface 208A of the backing layer 208 is configured to face away from the skin of the user. The proximal surface 208B of the backing layer 208 is covering the second adhesive layer 202.

The base plate 4 forms part of a two-piece ostomy appliance, thus comprising a coupling ring 209 for coupling an ostomy pouch to the base plate 4, such as to a distal side of the base plate 4.

The base plate 4 comprises a stomal opening. The layers of the base plate 4, such as the first adhesive layer 200, the second adhesive layer 202 and the backing layer 208 as illustrated, may comprise stomal openings 18 for collectively forming the stomal opening of the base plate.

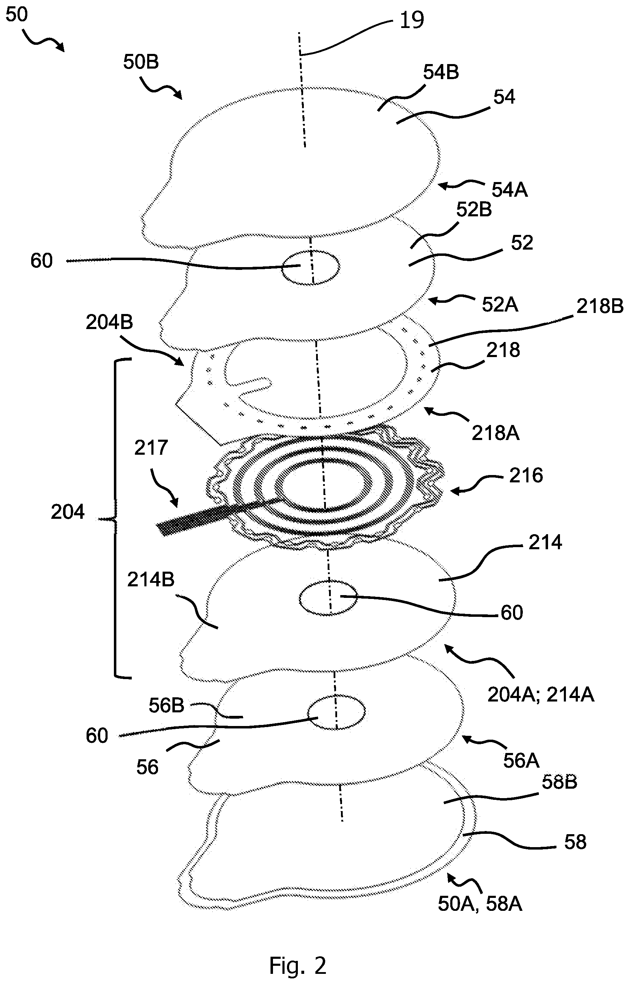

Fig. 2 schematically illustrates an exploded view of an exemplary two-piece ostomy appliance comprising a sensor patch 50, such as a sensor patch 50 being adapted for attachment to a base plate, such as the base plate 4 as illustrated in Fig. 1. The sensor patch 50 is configured to be positioned between the skin of the user and the proximal side of the base plate 4. For example, the sensor patch may be adapted for attachment to the first adhesive layer 200, such as the proximal surface 200B of the first adhesive layer 200, of the base plate 4 as illustrated in Fig. 1. The sensor patch 50 is configured to be attached to the base plate such that the distal side 50A of the sensor patch 50 is attached to the proximal side of the base plate, such as to the proximal surface 200B of the first adhesive layer 200 of the base plate 4.

The sensor patch 50 comprises a sensor assembly 204 comprising a plurality of electrode paths 216. Each electrode path 216 has respective connection parts 217 for connecting the plurality of electrode paths 216 to respective terminal elements of a monitor device. The sensor assembly 204 may form a sensor assembly layer.

The sensor assembly 204 has a distal side 204A and a proximal side 204B. The sensor assembly 204 comprises a support layer 214 with a proximal surface 214B. The electrode paths 216 may be provided, such as formed, on the proximal surface 214B of the support layer 214, e.g. the electrode paths 216 may be positioned on the proximal surface 214B of the support layer 214.

The electrode path assembly 204 comprises a masking element 218 having a distal surface 218A and a proximal surface 218B. The masking element 218 is configured to electrically insulate at least parts of electrode paths 216 from adjacent layers, such as the first adhesive sensor layer 52. The masking element 218 covers or overlaps with parts of the electrode paths 216 when seen in the axial direction.

The sensor patch 50 comprises a first adhesive sensor layer 52, with a proximal side 52B and a distal side 52A. The first adhesive sensor layer 52 is arranged on the proximal side 204B of the sensor assembly 204. The proximal side 52B of the first adhesive sensor layer 52 is configured to adhere to the user's skin. Thus, after being applied to the base plate, the combined base plate and sensor patch 50 forms an adhesive proximal surface configured to be applied to the skin surface of the user.

The sensor patch comprises a first sensor release liner 54. The first sensor release liner 54 may comprise a distal surface 54A and a proximal surface 54B. The first sensor release liner 54 may be arranged to protect the first adhesive sensor layer 52. The distal surface 54A of the first sensor release liner 54 is facing the proximal surface 52B of the first adhesive sensor layer 52. The first sensor release liner 54 is configured to be peeled off by the user prior to application of the base plate with the attached sensor patch to the skin. The first adhesive sensor layer 52 may be laid out on the distal side 54A of the first sensor release liner 54.

The sensor patch 50 comprises a second adhesive sensor layer 56, with a proximal side 56B and a distal side 56A. The second adhesive sensor layer 56 is arranged on the distal side 204A of the sensor assembly 204. The proximal side 56B of the second adhesive sensor layer 52 is configured to adhere to the base plate, such as the proximal surface of the first adhesive layer of the base plate.

The exemplary sensor patch comprises a second sensor release liner 58. The second sensor release liner 58 may comprise a distal surface 58A and a proximal surface 58B. The second sensor release liner 58 may be arranged to protect the second adhesive sensor layer 56. The proximal surface 58B of the second sensor release liner 58 is facing the distal surface 56A of the second adhesive sensor layer 56. The second sensor release liner 58 is configured to be peeled off by the user prior to application of the sensor patch to the base plate. The second adhesive sensor layer 56 may be laid out on the proximal side 58B of the second sensor release liner 58.

Whereas the illustrated exemplary sensor patch 50 comprises a second adhesive sensor layer 56 arranged on the distal side 204A of the sensor assembly 204, it is envisioned that an alternative sensor patch according to the disclosure may be provided without such a second adhesive sensor layer 56 and accompanying second sensor release liner 58. Rather, in such an alternative sensor patch, it is envisioned that the distal surface of the sensor patch is the distal surface of the sensor assembly 204, in particular the distal surface of the support layer 214. In such an alternative sensor patch, the distal surface of the support layer 214 may be configured for attachment to the adhesive surface of a base plate. Thus, whereas a sensor patch 50 having a first 52 and a second adhesive sensor layer 56 is illustrated and described, it is envisioned that a similar sensor patch without the second adhesive sensor layer 56 and accompanying second sensor release liner 58 may be provided according to the disclosure.

The sensor patch 50 comprises a stomal opening. The layers of the sensor patch 50, such as the first adhesive sensor layer 52, the support layer 214 and the second adhesive sensor layer 56, as illustrated, may comprise stomal openings 60 for collectively forming the stomal opening of the sensor patch 50. The stomal opening of the sensor patch is configured to be aligned with the stomal opening 18 of the base plate, such as to collectively form the stomal opening of the combined base plate and sensor patch 50.

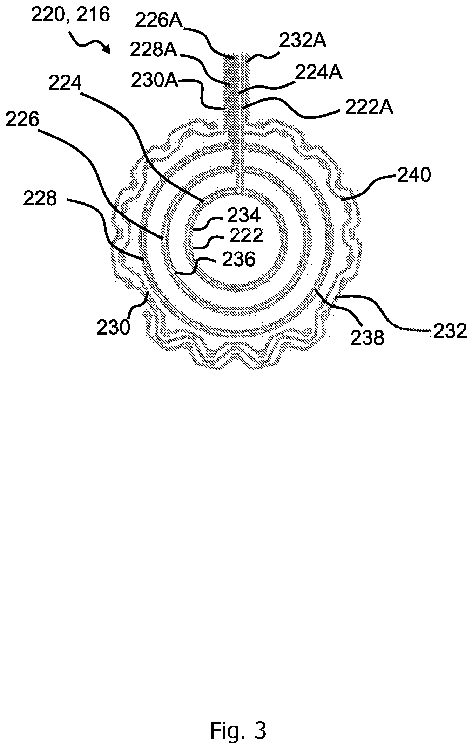

Fig. 3 schematically illustrates an exemplary electrode path configuration 220 of electrode paths 216 of an exemplary sensor assembly, such as the sensor assembly 204 as described with respect to Fig. 2. The plurality of electrode paths 216 comprises a first electrode path 222, a second electrode path 224, a third electrode path 226, a fourth electrode path 228, a fifth electrode path 230, and a sixth electrode path 232.

The first electrode path 222 comprises a first connection part 222A and the second electrode path 224 comprises a second connection part 224A. The third electrode path 226 comprises a third connection part 226A. The fourth electrode path 228 comprises a fourth connection part 228A. The fifth electrode path 230 comprises a fifth connection part 230A. The sixth electrode path 232 comprises a sixth connection part 232A.

The first electrode path 222 may be a common ground electrode, also denoted a reference electrode, such as to form sensors with respect to the remaining electrode paths. The first electrode path 222 comprises a first electrode part 234 for forming a ground for the second electrode path 224. The first electrode path 222 comprises a second electrode part 236 for forming a ground for the third electrode path 226. The first electrode path 222 comprises a third electrode part 238 for forming a ground for the fourth electrode path 228. The first electrode path 222 comprises a fourth electrode part 240 for forming a ground for the fifth electrode path 230 and the sixth electrode path 232.

As shown in Fig. 2, the sensor patch 50 comprises a stomal opening 60 with a centre point 19 (here illustrated as by a centre line). In some exemplary sensor patches, the stomal opening 60 may be made by the user, i.e. the sensor patch may be manufactured and/or sold without the stomal opening 60, but with a region adapted to form the stomal opening 60 with the centre point 19.

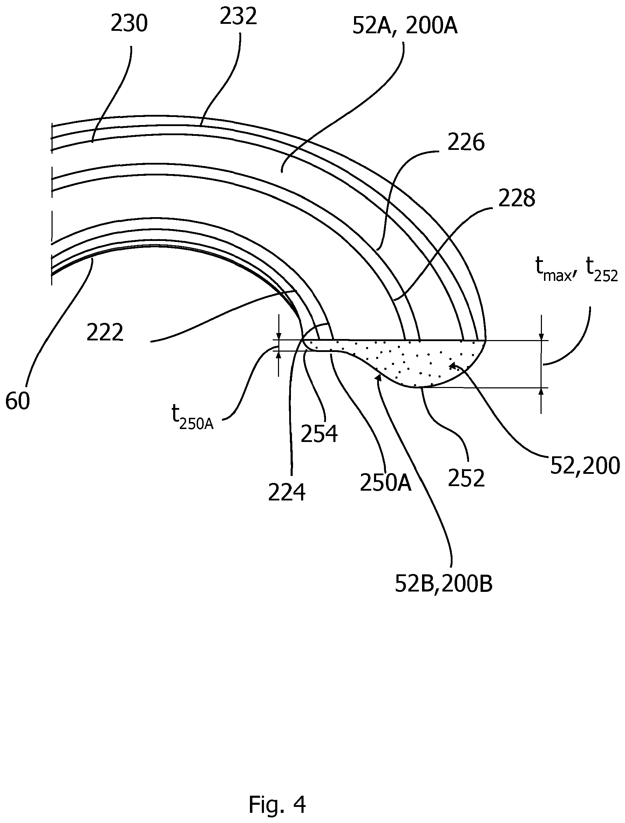

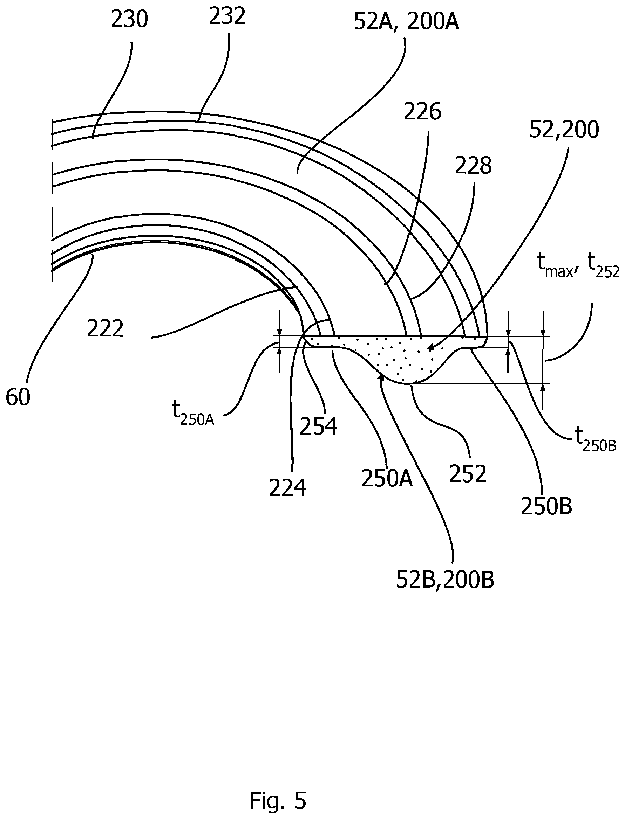

Fig. 4 schematically illustrates a perspective view of an exemplary adhesive layer 52, 200 and a plurality of electrode paths 222, 224, 226, 228, 230, 232 with a support layer omitted for visualization purposes. Fig. 4 shows about a quarter of the circumference of the adhesive layer and electrode paths around the stomal opening 60. The adhesive layer 52, 200 and the plurality of electrode paths 222, 224, 226, 228, 230, 232 are illustratively cut to show the cross-section. Fig. 5 schematically illustrates the same view as Fig. 4 but of an alternative embodiment of an adhesive layer 52, 200 and a plurality of electrode paths 222, 224, 226, 228, 230, 232. The shown adhesive layer 52, 200 and the plurality of electrode paths 222, 224, 226, 228, 230, 232 of Figs. 4-5 can form part of a base plate as shown in Fig. 1, in which case the backing layer 208 may form the support layer, or a sensor patch as shown in Fig. 2 that may form part of a one-piece or a two-piece ostomy appliance as described above. The plurality of electrode paths 222, 224, 226, 228, 230, 232 is arranged on the proximal surface of the visually omitted support layer. As seen, the adhesive layer 52, 200 includes a substantially flat distal surface 52A, 200A and a proximal surface 52B, 200B. The distal surface 52A, 200A is arranged on the proximal surface of the visually omitted support layer. The distal surface 52A, 200A covers the plurality of electrode paths (since the plurality of electrode paths are sandwiched between the shown distal surface 52A, 200A and the visually omitted support layer). Turning to Fig. 4, the adhesive layer 52, 200 comprises a first trough portion 250A covering a first electrode path 222 and a second electrode path 224. The first trough portion 250 extends circumferentially entirely around the stomal opening 60. In the shown embodiment, the adhesive layer 52, 200 comprises a rounded portion 254 extending around the stomal opening 60 and radially from the stomal opening to the first trough portion 250. The adhesive layer 52, 200 further comprises a crest portion 252 that extends circumferentially entirely around the stomal opening 60 and that has a thickness t₂₅₂ identical to a maximum thickness tₘₐₓ of the adhesive layer 52, 200. The crest portion 252 is radially distanced on an outer side of the first trough portion 252. The first trough portion 250A has a substantially uniform thickness t_{250A} which is less than a thickness t₂₅₂ of the crest portion 252. The crest portion 252 and the first trough portion 250A provide the proximal surface 52B, 200B of the adhesive layer 52, 200 with a ripple shape extending radially from the stomal opening 60. In the present embodiment, the thickness t₂₅₂ of the crest portion 252 is about 3 times greater than the thickness t_{250A} of the first trough portion 250A. The first and second electrode paths 222, 224 are configured for allowing the provision of a signal indicative of liquid on the proximal surface of the adhesive layer beneath the second trough portion 250B. Due to the relatively thin thickness of the first trough portion 250A, the first and second electrode paths 222, 224 have good electrical contact with the skin surface of the user and can function as an early warning sensor if a leak is initiated between the skin surface and the proximal surface 52B, 200B of the adhesive layer 52, 200. In other words, the relatively think thickness of the first trough portion 250A allows for a fast transfer of liquid (e.g., stomal output leaking between the skin and the ostomy appliance) from the proximal side, through the adhesive, and into contact with the electrode paths. Thereby, the proximal surface may be coherent/integral without the provision of openings exposing the electrodes, while at the same time facilitating a fast detection of liquid. The crest portion 252 covers a third and fourth electrode paths 226, 228 that may be configured for allowing the provision of a signal indicative of moisture content of the adhesive layer. Due to the relatively thick crest portion, the third and fourth electrode paths 226, 228 are arranged relatively far from the skin surface and can thus more reliably measure the moisture content of the adhesive layer. This allows inferring the time remaining before replacement of the base plate is advised. A fifth and a sixth electrode path 230, 232 are shown as well, arranged in the vicinity of the crest portion 252.

The relative thick thickness of the crest portion 252 allows for a large moisture absorbing capacity, such that the ostomy appliance may be used/worn for a long time, without compromising a fast detection of liquid, as facilitated by the trough portion 250A as explained above. In alternative embodiments, the third and fourth electrode paths 226, 228 and/or the fifth and sixth electrode paths 230, 232 may be omitted, such that the detection of liquid/moisture is facilitated by the first and second electrode paths 222, 224 only.

Fig. 5, as discussed, shows an alternative shape of the proximal surface 52B, 200B, of the adhesive layer 52, 200. In this embodiment, the adhesive layer comprises a second trough portion 250B extending around the stomal opening 60. The second trough portion covers a fifth electrode path 230 and a sixth electrode path 232. The thickness of the second trough portion 250B is substantially the same as the thickness of the first trough portion 250A. The fifth and sixth electrode paths 230, 232 are configured for allowing the provision of a signal indicative of liquid on the proximal surface of the adhesive layer beneath the second trough portion 250B. Accordingly, fifth and sixth electrode paths 230, 232 can function as a last warning sensor of when a leak is imminent. The crest portion 252 is arranged between the first trough portion 250A and the second trough portion 250B.
- 4: base plate
- 18: stomal opening
- 19: centre point
- 50: sensor patch
- 50A: distal side
- 52: first adhesive sensor layer
- 52B: proximal side
- 52A: distal side
- 54: first sensor release liner
- 54A: distal surface
- 54B: proximal surface
- 56: second adhesive sensor layer
- 56B: proximal side
- 56A: distal side
- 58: second sensor release liner
- 58A: distal surface
- 58B: proximal surface
- 60: stomal opening
- 200: first adhesive layer
- 200A: distal surface
- 200B: proximal surface
- 202: second adhesive layer
- 202A: distal surface
- 202B: proximal surface
- 204: sensor assembly
- 204A: distal side
- 204B: proximal side
- 206: release liner
- 206A: distal surface
- 206B: proximal surface
- 208: backing layer
- 208A: distal surface
- 208B: proximal surface
- 209: coupling ring
- 214: support layer
- 214A: distal surface
- 214B: proximal surface
- 216: electrode path
- 217: connection part
- 218: masking element
- 218A: distal surface
- 218B: proximal surface
- 220: electrode path configuration
- 222: first electrode path
- 222A: first connection part
- 224: second electrode path
- 224A: second connection part
- 226: third electrode path
- 226A: third connection part
- 228: fourth electrode path
- 228A: fourth connection part
- 230: fifth electrode path
- 230A: fifth connection part
- 232: sixth electrode path
- 232A: sixth connection part
- 234: first electrode part
- 236: second electrode part
- 238: third electrode part
- 240: fourth electrode part
- 250A: first trough portion
- t_{250A}: thickness
- 250B: second trough portion
- t_{250B}: thickness
- 252: crest portion
- t₂₅₂: thickness
- tₘₐₓ: maximum thickness
- 254: rounded portion

## Claims

1. An ostomy appliance for attachment to the skin surface of a user, wherein the ostomy appliance is adapted to form a stomal opening (18) with a centre point (19), the stomal opening being configured to allow passage of output through the stomal opening and into an ostomy pouch attached to the ostomy appliance, the ostomy appliance comprising:
- a support layer (214) with a distal surface (214A) and a proximal surface (214B);
- a plurality of electrode paths (220) for forming one or more sensors, the plurality of electrode paths being arranged on the proximal surface (214B) of the support layer (214); and
- an adhesive layer (200, 52) including a distal surface (200A, 52A) and a proximal surface (200B, 52B), the distal surface (200A) being arranged on the proximal surface (214B) of the support layer (214) and covering the plurality of electrode paths (220), wherein the adhesive layer comprises one or more trough portions (250A) covering a first electrode path (222) and a second electrode path (224) of the plurality of electrode paths, and a crest portion (252) comprising a maximum thickness (tₘₐₓ) of the adhesive layer (200, 52) and being distanced from the one or more trough portions (250A),
**characterised in that** the one or more trough portions has a substantially uniform thickness (t_{250A}), wherein the substantially uniform thickness (t_{250A}) of the one or more trough portions (250A) is less than a thickness (t₂₅₂) of the crest portion (252), and **in that** the one or more trough portions extends circumferentially at least partly around the stomal opening, and **in that** the crest portion extends circumferentially at least partly around the stomal opening.

2. An ostomy appliance according to claim 1, wherein the distal surface of the adhesive layer is substantially flat and arranged on the proximal surface of the support layer, wherein the crest portion and the one or more trough portions at least partly provide the proximal surface of the adhesive layer with a ripple shape extending radially from the centre point.

3. An ostomy appliance according to any one of the previous claims, wherein the crest portion is arranged on a radially outer side of the one or more trough portions.

4. An ostomy appliance according to any one of the claims 1-2, wherein the crest portion is arranged on a radially inner side of the one or more trough portions.

5. An ostomy appliance according to any one of claims 1-2, wherein the one or more trough portions include a first trough portion (250A) and a second trough portion (250B), and wherein the crest portion is arranged between the first trough portion (250A) and the second trough portion (250B), the first trough portion covering the first electrode path and the second trough portion covering the second electrode path.

6. An ostomy appliance according to any one of the previous claims, wherein the plurality of electrode paths extends circumferentially at least partly around the stomal opening.

7. An ostomy appliance according to any one of the previous claims, wherein the thickness of the one or more trough portions is at most 150 µm.

8. An ostomy appliance according to any one of the previous claims, wherein the thickness of the crest portion is at least 80 µm.

9. An ostomy appliance according to any one of the previous claims, wherein the difference between the thickness of the one or more trough portions and the thickness of the crest portion is at least 30 µm.

10. An ostomy appliance according to any one of the previous claims, wherein the first and second electrode paths are configured for allowing the provision of a signal indicative of liquid on the proximal surface of the adhesive layer.

11. An ostomy appliance according to any one of the previous claims, wherein the plurality of electrode paths further includes a third and a fourth electrode path (226, 228) configured for allowing the provision of a signal indicative of the moisture content of the adhesive layer.

12. An ostomy appliance according to any one of the previous claims, wherein a first trough portion of the one or more trough portions is delimited by a radially innermost electrode path and a radially outermost electrode path of the plurality of electrode paths.

13. An ostomy appliance according to any one of the previous claims, wherein the ostomy appliance comprises a base plate (4), the base plate including the support layer, the plurality of electrode paths, and the adhesive layer.

14. An ostomy appliance according to any one of the claims 1-12, wherein the ostomy appliance comprises a sensor patch (50) having a distal side (50A) and a proximal side, the distal side (50A) being adapted for attachment to an adhesive surface of a base plate (4) of the ostomy appliance, and the proximal side being adapted for attachment to a skin surface of a user, the sensor patch including the support layer, the plurality of electrode paths, and the adhesive layer.

15. A method of manufacturing an ostomy appliance according to any one of the previous claims, comprising the steps of:
- providing a support layer (214) with a distal surface (214A) and a proximal surface (214B);
- arranging a plurality of electrode paths (220) on the proximal surface (214B) of the support layer (214);
- arranging an adhesive layer (200, 52) on the proximal surface (214B) of the support layer (214) so as to cover the plurality of electrode paths (220), wherein the adhesive layer (200, 52) comprises one or more trough portions (250A) covering a first electrode path (222) and a second electrode path (224) of the plurality of electrode paths (220), and a crest portion (252) comprising a maximum thickness of the adhesive layer (200, 52) and being distanced from the one or more trough portions (250A),
**characterised in that** the one or more trough portions (250A) has a substantially uniform thickness (t_{250A}), wherein the substantially uniform thickness of the one or more trough portions (250A) is less than the thickness of the crest portion (252), and **in that** the one or more trough portions (250A) extends circumferentially at least partly around the stomal opening (18), and **in that** the crest portion (252) extends circumferentially at least partly around the stomal opening (18).

## Patentansprüche

1. Stomavorrichtung zur Befestigung an der Hautoberfläche eines Benutzers, wobei die Stomavorrichtung dafür ausgelegt ist, eine Stomaöffnung (18) mit einem Mittelpunkt (19) zu bilden, wobei die Stomaöffnung dazu ausgelegt ist, den Durchgang einer Ausscheidung durch die Stomaöffnung und in einen an der Stomavorrichtung befestigten Stomabeutel zu ermöglichen, wobei die Stomavorrichtung umfasst:
- eine Trägerschicht (214) mit einer distalen Oberfläche (214A) und einer proximalen Oberfläche (214B);
- eine Mehrzahl von Elektrodenbahnen (220) zum Bilden eines oder mehrerer Sensoren, wobei die Mehrzahl von Elektrodenbahnen auf der proximalen Oberfläche (214B) der Trägerschicht (214) angeordnet ist; und
- eine Klebeschicht (200, 52), die eine distale Oberfläche (200A, 52A) und eine proximale Oberfläche (200B, 52B) aufweist, wobei die distale Oberfläche (200A) auf der proximalen Oberfläche (214B) der Trägerschicht (214) angeordnet ist und die Mehrzahl von Elektrodenbahnen (220) bedeckt, wobei die Klebeschicht einen oder mehrere Muldenabschnitte (250A), die eine erste Elektrodenbahn (222) und eine zweite Elektrodenbahn (224) der Mehrzahl von Elektrodenbahnen bedecken, und einen Kammabschnitt (252), der eine maximale Dicke (tₘₐₓ) der Klebeschicht (200, 52) umfasst und von dem einen oder den mehreren Muldenabschnitten (250A) beabstandet ist, aufweist,
**dadurch gekennzeichnet, dass** der eine oder die mehreren Muldenabschnitte eine im Wesentlichen gleichmäßige Dicke (t_{250A}) aufweisen, wobei die im Wesentlichen gleichmäßige Dicke (t_{250A}) des einen oder der mehreren Muldenabschnitte (250A) geringer als eine Dicke (t₂₅₂) des Kammabschnitts (252) ist, und dass sich der eine oder die mehreren Muldenabschnitte in Umfangsrichtung zumindest teilweise um die Stomaöffnung erstrecken und dass sich der Kammabschnitt in Umfangsrichtung zumindest teilweise um die Stomaöffnung erstreckt.

2. Stomavorrichtung nach Anspruch 1, wobei die distale Oberfläche der Klebeschicht im Wesentlichen flach ist und auf der proximalen Oberfläche der Trägerschicht angeordnet ist, wobei der Kammabschnitt und der eine oder die mehreren Muldenabschnitte die proximale Oberfläche der Klebeschicht zumindest teilweise mit einer Wellenform versehen, die sich radial aus dem Mittelpunkt erstreckt.

3. Stomavorrichtung nach einem der vorhergehenden Ansprüche, wobei der Kammabschnitt auf einer radial äußeren Seite des einen oder der mehreren Muldenabschnitte angeordnet ist.

4. Stomavorrichtung nach einem der Ansprüche 1-2, wobei der Kammabschnitt auf einer radial inneren Seite des einen oder der mehreren Muldenabschnitte angeordnet ist.

5. Stomavorrichtung nach einem der Ansprüche 1-2, wobei der eine oder die mehreren Muldenabschnitte einen ersten Muldenabschnitt (250A) und einen zweiten Muldenabschnitt (250B) umfassen und wobei der Kammabschnitt zwischen dem ersten Muldenabschnitt (250A) und dem zweiten Muldenabschnitt (250B) angeordnet ist, wobei der erste Muldenabschnitt die erste Elektrodenbahn bedeckt und der zweite Muldenabschnitt die zweite Elektrodenbahn bedeckt.

6. Stomavorrichtung nach einem der vorhergehenden Ansprüche, wobei sich die Mehrzahl von Elektrodenbahnen in Umfangsrichtung zumindest teilweise um die Stomaöffnung herum erstreckt.

7. Stomavorrichtung nach einem der vorhergehenden Ansprüche, wobei die Dicke des einen oder der mehreren Muldenabschnitte höchstens 150 µm beträgt.

8. Stomavorrichtung nach einem der vorhergehenden Ansprüche, wobei die Dicke des Kammabschnitts mindestens 80 µm beträgt.

9. Stomavorrichtung nach einem der vorhergehenden Ansprüche, wobei die Differenz zwischen der Dicke des einen oder der mehreren Muldenabschnitte und der Dicke des Kammabschnitts mindestens 30 µm beträgt.

10. Stomavorrichtung nach einem der vorhergehenden Ansprüche, wobei die erste und die zweite Elektrodenbahn dazu ausgelegt sind, das Bereitstellen eines Signals, das Flüssigkeit auf der proximalen Oberfläche der Klebeschicht angibt, zu ermöglichen.

11. Stomavorrichtung nach einem der vorhergehenden Ansprüche, wobei die Mehrzahl von Elektrodenbahnen ferner eine dritte und eine vierte Elektrodenbahn (226, 228) umfasst, die dazu ausgelegt sind, das Bereitstellen eines Signals zu ermöglichen, das den Feuchtigkeitsgehalt der Klebeschicht angibt.

12. Stomavorrichtung nach einem der vorhergehenden Ansprüche, wobei ein erster Muldenabschnitt des einen oder der mehreren Muldenabschnitte durch eine radial innerste Elektrodenbahn und eine radial äußerste Elektrodenbahn der Mehrzahl von Elektrodenbahnen begrenzt ist.

13. Stomavorrichtung nach einem der vorhergehenden Ansprüche, wobei die Stomavorrichtung eine Basisplatte (4) umfasst, wobei die Basisplatte die Trägerschicht, die Mehrzahl von Elektrodenbahnen und die Klebeschicht umfasst.

14. Stomavorrichtung nach einem der Ansprüche 1-12, wobei die Stomavorrichtung ein Sensorpflaster (50) mit einer distalen Seite (50A) und einer proximalen Seite umfasst, wobei die distale Seite (50A) zur Befestigung an einer Klebeoberfläche einer Basisplatte (4) der Stomavorrichtung ausgelegt ist und wobei die proximale Seite zur Befestigung an einer Hautoberfläche eines Benutzers ausgelegt ist, wobei das Sensorpflaster die Trägerschicht, die Mehrzahl von Elektrodenbahnen und die Klebeschicht umfasst.

15. Verfahren zum Fertigen einer Stomavorrichtung nach einem der vorhergehenden Ansprüche, das die folgenden Schritte umfasst:
- Bereitstellen einer Trägerschicht (214) mit einer distalen Oberfläche (214A) und einer proximalen Oberfläche (214B);
- Anordnen einer Mehrzahl von Elektrodenbahnen (220) auf der proximalen Oberfläche (214B) der Trägerschicht (214).
- Anordnen einer Klebeschicht (200, 52) auf der proximalen Oberfläche (214B) der Trägerschicht (214) so, dass sie die Mehrzahl von Elektrodenbahnen (220) bedeckt, wobei die Klebeschicht (200, 52) einen oder mehrere Muldenabschnitte (250A), die eine erste Elektrodenbahn (222) und eine zweite Elektrodenbahn (224) der Mehrzahl von Elektrodenbahnen (220) bedecken, und einen Kammabschnitt (252), der eine maximale Dicke der Klebeschicht (200, 52) umfasst und von dem einen oder den mehreren Muldenabschnitten (250A) beabstandet ist, aufweist,
**dadurch gekennzeichnet, dass** der eine oder die mehreren Muldenabschnitte (250A) eine im Wesentlichen gleichmäßige Dicke (t_{250A}) aufweisen, wobei die im Wesentlichen gleichmäßige Dicke des einen oder der mehreren Muldenabschnitte (250A) geringer als die Dicke des Kammabschnitts (252) ist, und dass sich der eine oder die mehreren Muldenabschnitte (250A) in Umfangsrichtung zumindest teilweise um die Stomaöffnung (18) herum erstrecken, und dass sich der Kammabschnitt (252) in Umfangsrichtung zumindest teilweise um die Stomaöffnung (18) herum erstreckt.

## Revendications

1. Appareil de stomie destiné à être fixé à la surface cutanée d'un utilisateur, l'appareil de stomie étant adapté pour former une ouverture stomale (18) avec un point central (19), l'ouverture stomale étant configurée pour permettre le passage de la sortie à travers l'ouverture stomale et dans une poche de stomie fixée à l'appareil de stomie, l'appareil de stomie comprenant :
- une couche de support (214) avec une surface distale (214A) et une surface proximale (214B) ;
- une pluralité de chemins d'électrode (220) pour former un ou plusieurs capteurs, la pluralité de chemins d'électrode étant agencée sur la surface proximale (214B) de la couche de support (214) ; et
- une couche adhésive (200, 52) comprenant une surface distale (200A, 52A) et une surface proximale (200B, 52B), la surface distale (200A) étant disposée sur la surface proximale (214B) de la couche de support (214) et recouvrant la pluralité de chemins d'électrode (220), dans lequel la couche adhésive comprend une ou plusieurs parties en creux (250A) recouvrant un premier chemin d'électrode (222) et un deuxième chemin d'électrode (224) de la pluralité de chemins d'électrode, et une partie de crête (252) comprenant une épaisseur maximale (tₘₐₓ) de la couche adhésive (200, 52) et étant à distance de la ou des parties en creux (250A),
**caractérisé en ce que** la ou les parties en creux ont une épaisseur sensiblement uniforme (t_{250A}), l'épaisseur sensiblement uniforme (t_{250A}) de la ou des parties en creux (250A) étant inférieure à une épaisseur (t₂₅₂) de la partie de crête (252), et **en ce que** la ou les parties en creux s'étendent circonférentiellement au moins partiellement autour de l'ouverture stomale, et **en ce que** la partie de crête s'étend circonférentiellement au moins partiellement autour de l'ouverture stomale.

2. Appareil de stomie selon la revendication 1, dans lequel la surface distale de la couche adhésive est sensiblement plate et agencée sur la surface proximale de la couche de support, dans lequel la partie de crête et la ou les parties en creux confèrent au moins partiellement à la surface proximale de la couche adhésive une forme ondulée s'étendant radialement à partir du point central.

3. Appareil de stomie selon l'une quelconque des revendications précédentes, dans lequel la partie de crête est agencée sur un côté radialement extérieur de la ou des parties en creux.

4. Appareil de stomie selon l'une quelconque des revendications 1 à 2, dans lequel la partie de crête est agencée sur un côté radialement intérieur de la ou des parties en creux.

5. Appareil de stomie selon l'une quelconque des revendications 1 à 2, dans lequel la ou les parties en creux comprennent une première partie en creux (250A) et une seconde partie en creux (250B), et dans lequel la partie de crête est agencée entre la première partie en creux (250A) et la seconde partie en creux (250B), la première partie en creux recouvrant le premier chemin d'électrode et la seconde partie en creux recouvrant le deuxième chemin d'électrode.

6. Appareil de stomie selon l'une quelconque des revendications précédentes, dans lequel la pluralité de chemins d'électrodes s'étend circonférentiellement au moins partiellement autour de l'ouverture stomale.

7. Appareil de stomie selon l'une quelconque des revendications précédentes, dans lequel l'épaisseur de la ou des parties en creux est de 150 µm au plus.

8. Appareil de stomie selon l'une quelconque des revendications précédentes, dans lequel l'épaisseur de la partie de crête est d'au moins 80 µm.

9. Appareil de stomie selon l'une quelconque des revendications précédentes, dans lequel la différence entre l'épaisseur de la ou des parties en creux et l'épaisseur de la partie de crête est d'au moins 30 µm.

10. Appareil de stomie selon l'une quelconque des revendications précédentes, dans lequel les premier et deuxième chemins d'électrode sont configurés pour permettre la fourniture d'un signal indicatif d'un liquide sur la surface proximale de la couche adhésive.

11. Appareil de stomie selon l'une quelconque des revendications précédentes, dans lequel la pluralité de chemins d'électrodes comprend en outre un troisième et un quatrième chemins d'électrodes (226, 228) configurés pour permettre la fourniture d'un signal indicatif de la teneur en humidité de la couche adhésive.

12. Appareil de stomie selon l'une quelconque des revendications précédentes, dans lequel une première partie en creux de la ou des parties en creux est délimitée par un chemin d'électrode radialement le plus intérieur et un chemin d'électrode radialement le plus extérieur de la pluralité de chemins d'électrode.

13. Appareil de stomie selon l'une quelconque des revendications précédentes, dans lequel l'appareil de stomie comprend une plaque de base (4), la plaque de base comprenant la couche de support, la pluralité de chemins d'électrode et la couche adhésive.

14. Appareil de stomie selon l'une quelconque des revendications 1 à 12, l'appareil de stomie comprenant un timbre de détection (50) ayant un côté distal (50A) et un côté proximal, le côté distal (50A) étant adapté pour être fixé à une surface adhésive d'une plaque de base (4) de l'appareil de stomie, et le côté proximal étant adapté pour être fixé à une surface cutanée d'un utilisateur, le timbre de détection comportant la couche de support, la pluralité de chemins d'électrode et la couche adhésive.

15. Procédé de fabrication d'un appareil de stomie selon l'une quelconque des revendications précédentes, comprenant les étapes suivantes :
- la fourniture d'une couche de support (214) ayant une surface distale (214A) et une surface proximale (214B) ;
- l'agencement d'une pluralité de chemins d'électrodes (220) sur la surface proximale (214B) de la couche de support (214) ;
- la disposition d'une couche adhésive (200, 52) sur la surface proximale (214B) de la couche de support (214) de manière à recouvrir la pluralité de chemins d'électrode (220), dans lequel la couche adhésive (200, 52) comprend une ou plusieurs parties en creux (250A) recouvrant un premier chemin d'électrode (222) et un deuxième chemin d'électrode (224) de la pluralité de chemins d'électrode (220), et une partie de crête (252) comprenant une épaisseur maximale de la couche adhésive (200, 52) et étant à distance de la ou des parties en creux (250A),
**caractérisé en ce que** la ou les parties en creux (250A) ont une épaisseur sensiblement uniforme (t_{250A}), l'épaisseur sensiblement uniforme de la ou des parties en creux (250A) étant inférieure à l'épaisseur de la partie de crête (252), et **en ce que** la ou les parties en creux (250A) s'étendent circonférentiellement au moins partiellement autour de l'ouverture stomale (18), et **en ce que** la partie de crête (252) s'étend circonférentiellement au moins partiellement autour de l'ouverture stomale (18).
